# EUROPÄISCHE PATENTSCHRIFT

(11) **EP 4 175 567 B1**
(45) Veröffentlichungstag und Bekanntmachung des Hinweises auf die Patenterteilung: **02.04.2025**
(21) Anmeldenummer: 21746651.5
(22) Anmeldetag: 05.07.2021
(51) Int. Cl.: A61B 17/22

(54) **LITHOTRIPSIEVORRICHTUNG UND VERFAHREN ZUM TESTBETRIEB EINER LITHOTRIPSIEVORRICHTUNG**
LITHOTRIPSY DEVICE, AND METHOD FOR TEST OPERATING A LITHOTRIPSY DEVICE
DISPOSITIF DE LITHOTRIPSIE ET PROCÉDÉ DE FONCTIONNEMENT EN MODE TEST D'UN DISPOSITIF DE LITHOTRIPSIE

(30) Priorität: 06.07.2020 DE 102020117713
(43) Veröffentlichungstag der Anmeldung: 10.05.2023
(73) Patentinhaber: Karl Storz SE & Co. KG, 78532 Tuttlingen (DE)
(72) Erfinder: GLÖGGLER, Bernhard, 78532 Tuttlingen (DE); GÖBEL, Werner, 78532 Tuttlingen (DE); HINDING, Thomas, 78532 Tuttlingen (DE); HUBER, Florian, 78532 Tuttlingen (DE)
(86) Internationale Anmeldenummer: PCT/EP2021/068510
(87) Internationale Veröffentlichungsnummer: WO 2022/008440

(56) Entgegenhaltungen:
- EP-A1- 1 163 882
- EP-A1- 1 163 883
- WO-A1-2019/141822

## Beschreibung

### Stand der Technik

Die Erfindung betrifft eine Lithotripsievorrichtung, insbesondere eine intrakorporale Lithotripsievorrichtung, nach dem Oberbegriff des Anspruchs 1 sowie ein Verfahren zum extrakorporalen Testbetrieb einer Lithotripsievorrichtung nach dem Oberbegriff des Anspruchs 19. Chirurgische oder therapeutische Verfahren werden nicht beansprucht.

Aus dem Stand der Technik ist bereits eine Lithotripsievorrichtung bekannt, welche eine Sonotrode, welche einen Sonotrodenkopf und eine Sonotrodenspitze umfasst, und ein Ultraschallhorn aufweist. Das Ultraschallhorn ist dazu eingerichtet, entlang einer Übertragungsrichtung Ultraschallwellen auf den Sonotrodenkopf zu übertragen und zu fokussieren. Ferner ist die Sonotrode dazu eingerichtet, die Ultraschallwellen entlang derselben Übertragungsrichtung die Ultraschallwellen von dem Sonotrodenkopf zu der Sonotrodenspitze zu leiten.

EP 1 163 883 A1 beansprucht eine Vorrichtung zum Entfernen von Körpersteinen mit einem intrakorporalen Lithotripter, bei dem eine als Wellenleiter ausgebildete Metallsonde mittels eines elektrisch angesteuerten Ultraschallwandlers zur longitudinalen Schwingung angeregt und diese Schwingungsanregung auf eine mit einem reversibel angetriebenen Schlagteil gesteuerte Ausbildung einer Stoß- beziehungsweise Druckwelle in der Metallsonde umschaltbar ist. Hierbei werden die erzeugten Ultraschallschwingungen vom Horn des Ultraschallwandlers in distaler Richtung fokussiert und von der Metallsonde weiter in distaler Richtung bis zur Sondenspitze zum Entfernen von Körpersteinen übertragen.

In der EP 1 163 882 A1 wird eine Vorrichtung zum Entfernen von Körpersteinen mit einem intrakorporalen Lithotripter beschrieben, bei dem eine Sonotrode mittels eines Ultraschallwandlers zur longitudinalen Schwingung anregbar ist und proximalseitig der hohlen Sonotrode eine axial fluchtende Führungshülse für ein reversibel angetriebenes Schlagteil angeordnet ist, welches auf einen Massekörper einer Stoßsonde einwirkbar ist, welche in dem Hohlraum der Sonotrode aufgenommen ist. Hierbei erfolgen die Schwingungsanregung und die Stoßanregung ebenfalls in dieselbe, distale Richtung.

WO 2019/141822 A1 betrifft eine Vorrichtung zum Zertrümmern von Körpersteinen mit einer Sonde und einer Antriebseinheit zur Auslenkung der Sonde entlang ihrer Längserstreckung, wobei die Antriebseinheit eine erste Antriebseinrichtung zur periodischen Auslenkung der Sonde und eine zweite Antriebseinrichtung zur impulsförmigen Ausrichtung der Sonde aufweist, wobei die Übertragung der periodischen Auslenkung und der impulsförmigen Auslenkung beide in Richtung der Längsachse der Sonotrode zur Sonotrodenspitze erfolgt.

Die Aufgabe der Erfindung besteht insbesondere darin, eine gattungsgemäße Vorrichtung mit verbesserten Eigenschaften hinsichtlich einer Effizienz, insbesondere einer Bauraumeffizienz und/oder einer Bauteileffizienz bereitzustellen. Die Aufgabe wird erfindungsgemäß durch eine Vorrichtung mit den Merkmalen des Patentanspruchs 1 bzw. einem Verfahren mit den Merkmalen gemäß Patentanspruch 19 gelöst, während vorteilhaft Ausgestaltung und Weiterbildung der Erfindung den Unteransprüchen entnommen werden können.

### Vorteile der Erfindung

Die Erfindung geht aus von einer Lithotripsievorrichtung, insbesondere einer intrakorporalen Lithotripsievorrichtung, mit wenigstens einer Sonotrode, welche zur Aufnahme von Ultraschallwellen wenigstens einen Sonotrodenkopf und zur Beaufschlagung von Konkrementen wenigstens eine Sonotrodenspitze, welche mit dem Sonotrodenkopf verbunden ist, umfasst, und mit einem Ultraschallhorn.

Es wird vorgeschlagen, dass das Ultraschallhorn dazu eingerichtet ist, entlang einer ersten Übertragungsrichtung Ultraschallwellen zumindest mittelbar auf den Sonotrodenkopf zu übertragen und zu fokussieren, und die Sonotrode dazu eingerichtet ist, entlang wenigstens einer zweiten Übertragungsrichtung, die von der ersten Übertragungsrichtung verschieden ist, die Ultraschallwellen vom Sonotrodenkopf zur Sonotrodenspitze zu übertragen.

Hierdurch kann vorteilhaft eine Effizienz verbessert werden. Insbesondere kann eine kompakte Anordnung von Bauteilen realisiert werden, wodurch sich weitere Baugruppen und Komponenten eines Lithotripters, wie insbesondere eine Stoßimpulseinheit, zur kombinierten Lithotripsie einbinden lassen. Besonders vorteilhaft kann eine Energieübertragung von Energie, bereitstellenden Komponenten auf die Sonotrode verbessert werden, wie beispielsweise in Form von Ultraschallwellen und/oder Stoßimpulsen. Besonders vorteilhaft kann ein modularer Aufbau erzielt werden, welcher je nach Kombination bzw. Betrieb einzelner Module, sowohl den Einzelbetrieb nur mit Ultraschallwellen oder einen Kombinationsbetrieb mit Ultraschallwellen und Stoßimpulsen erlaubt. Dies kann insbesondere zur Beseitigung von besonders großen oder festen Konkrementen von Vorteil sein.

Unter einer "Lithotripsievorrichtung" soll insbesondere ein vorzugsweise funktionsfähiger Bestandteil, beispielsweise einer Unterbaugruppe und/oder eine Konstruktions- und/oder eine Funktionskomponente eines Lithotripters verstanden werden. Vorzugsweise kann die Lithotripsievorrichtung den Lithotripter zumindest teilweise, bevorzugt zumindest zu einem Großteil und besonders bevorzugt vollständig ausbilden. Die Lithotripsievorrichtung ist insbesondere zu einer Zertrümmerung von Konkrementen eingerichtet. Unter "Konkrementen" sollen insbesondere sich in Körpern durch Abscheidung entstehende feste Gebilde verstanden werden, welche zumindest teilweise, vorzugsweise zumindest zu einem Großteil und besonders bevorzugt vollständig aus Salzen bestehen, wie beispielsweise Nierensteine, Gallensteine, Harnsteine, Speichelsteine oder dergleichen. Unter einer "intrakorporalen Lithotripsievorrichtung" soll insbesondere eine Lithotripsievorrichtung verstanden werden, welche dazu eingerichtet ist zumindest teilweise und vorzugsweise zumindest zu einem Großteil in eine insbesondere künstliche und/oder natürliche Öffnung, insbesondere Körperöffnung, eingeführt zu werden, um dort angesammelte Konkremente zu zertrümmern und/oder zu entfernen. Unter "eingerichtet" soll insbesondere speziell programmiert, vorgesehen, ausgelegt, ausgebildet und/oder ausgestattet verstanden werden. Darunter, dass ein Bauteil zu einer bestimmten Funktion eingerichtet ist, soll insbesondere verstanden werden, dass das Bauteil diese bestimmte Funktion in zumindest einen Anwendungs- und/oder Betriebszustand erfüllt und/oder ausführt. Unter dem Ausdruck "zumindest zu einem Großteil" soll dabei insbesondere dabei zumindest zu 55%, vorzugsweise zumindest zu 65%, bevorzugst zumindest zu 70% und besonders bevorzugt zumindest zu 85% und ganz besonders bevorzugt zumindest zu 95%, sowie vorteilhaft vollständig verstanden werden und zwar insbesondere mit Bezug auf ein Volumen und/oder eine Masse eines Bauteilts. Unter einer "Sonotrode" soll insbesondere eine stab-, röhren- und/oder schlauchförmige Sonde verstanden werden, welche zur Untersuchung und/oder Behandlung eingerichtet ist. Vorzugsweise ist die Sonotrode als eine intrakorporale Sonotrode ausgebildet. Unter einer "intrakorporalen Sonotrode" soll insbesondere eine Sonotrode verstanden werden, welche dazu eingerichtet ist, zumindest teilweise, vorzugsweise zumindest zu einem Großteil und besonders bevorzugt vollständig in eine insbesondere künstliche und/oder natürliche Öffnung, insbesondere Körperöffnung, eingeführt zu werden, um beispielsweise dort angesammelte Konkremente zu zertrümmern und/oder zu entfernen. Die Sonotrode weist insbesondere zumindest eine Sonotrodenspitze auf, welche dazu eingerichtet ist, Konkremente unmittelbar zu kontaktieren sowie Ultraschallwellen auf diese zu übertragen. Anstelle einer unmittelbaren Beaufschlagung kann auch eine zumindest mittelbare Beaufschlagung der Konkremente durch die Sonotrode denkbar sein. Unter "zumindest mittelbar" soll insbesondere indirekt, aber vorzugsweise auch direkt, also insbesondere unmittelbar, verstanden werden. Der Sonotrodenkopf und die Sonotrodenspitze der Sonotrode sind vorzugsweise voneinander separat ausgebildet. Alternativ könnten Sonotrodenkopf und Sonotrodenspitze einstückig ausgebildet und/oder verbunden sein. Darunter, dass "ein Bauteil und ein weiteres Bauteil zumindest teilweise einstückig ausgebildet und/oder verbunden sind", soll insbesondere verstanden werden, dass zumindest ein Element und/oder Teil des Bauteils und zumindest ein Element und/oder ein Teil des weiteren Bauteils einteilig miteinander ausgebildet und/oder verbunden sind. Unter "einstückig" soll insbesondere zumindest stoffschlüssig verbunden, wie beispielsweise durch einen Schweißprozess, einen Klebeprozess, einen Anspritzprozess und/oder einen anderen dem Fachmann als sinnvoll erscheinenden Prozess und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Die Lithotripsievorrichtung weist insbesondere wenigstens einen Ultraschallgenerator auf. Der Ultraschallgenerator ist dazu eingerichtet, die Ultraschallwellen bereitzustellen und/oder zu erzeugen. Zur Erzeugung der Ultraschallwellen weist der Ultraschallgenerator wenigstens einen, vorzugsweise zumindest zwei, besonders bevorzugst zumindest drei und ganz besonders bevorzugt eine Vielzahl von Ultraschallaktoren auf. Der Ultraschallaktor ist vorzugsweise als ein Piezoaktor ausgebildet. Es sind jedoch auch andere, insbesondere mechanische Ausgestaltungen des Ultraschallaktors denkbar. Insbesondere für den Fall, dass der Ultraschallgenerator zumindest zwei Ultraschallaktoren umfasst, liegen diese zumindest mittelbar aneinander an, um vorteilhaft eine Intensität der erzeugten Ultraschallwellen zu erhöhen. Der Ultraschallgenerator ist insbesondere mit dem Ultraschallhorn wirkverbunden. Vorzugseise liegt das Ultraschallhorn zumindest mittelbar an dem Ultraschallgenerator an. Bevorzugt besteht das Ultraschallhorn zumindest teilweise, vorzugsweise zumindest zu einem Großteil und besonders bevorzugt vollständig aus Metall, wie beispielsweise Eisen, Titan, Stahl oder einer Metalllegion. Unter "Ultraschallwellen" sollen insbesondere Schallwellen verstanden werden, welche oberhalb eines für den Menschen wahrnehmbaren Hörfrequenzbereichs liegen. Im vorliegenden Fall weisen die Ultraschallwelle insbesondere eine Ultraschallfrequenz von zumindest 10 kHz, vorzugsweise zumindest 15 kHz und besonders bevorzugt von zumindest 20 kHz und/oder von höchstens 100 kHz, vorzugsweise von höchstens 80 kHz und besonders bevorzugt von höchstens 56 kHz auf. Ganz besonders bevorzugt beträgt die Ultraschallfrequenz der Ultraschallwellen einen Wert zwischen 22 kHz und 55 kHz. Unter einer "Übertragungsrichtung" soll insbesondere eine Richtung verstanden werden, welche eine Übertragung von Ultraschallwellen entlang eines Bauteils entspricht. Insbesondere ist die erste Übertragungsrichtung zumindest im Wesentlichen parallel zu einer Haupterstreckungsrichtung des Ultraschallhorn. Ferner ist insbesondere die zweite Übertragungsrichtung zumindest im Wesentlichen parallel zu einer Haupterstreckungsrichtung der Sonotrode. Unter einer "Haupterstreckungsrichtung" eines Bauteils soll dabei insbesondere eine Richtung verstanden werden, welche parallel zu einer längsten Kante eines kleinsten gedachten Quaders verläuft, welcher das Bauteil gerade noch vollständig umschließt. Unter "zumindest im Wesentlichen parallel" soll insbesondere das Einschließen eines Winkels von 0°verstanden werden, wobei insbesondere dieser Winkel eine Abweichung von höchstens 15°, vorzugsweise von höchstens 10° und besonders bevorzug von höchstens 5° aufweisen kann.

Es ist denkbar, dass die erste Übertragungsrichtung und die zweite Übertragungsrichtung winklig zueinander sind, um beispielsweise einen Schereffekt bei der Beaufschlagung der Konkremente zu erzeugen, wodurch vorteilhaft eine Zerstörung der Konkremente verbessert werden kann. Des Weiteren wird vorgeschlagen, dass die erste Übertragungsrichtung und die zweite Übertragungsrichtung einander entgegengerichtet sind. Hierdurch kann eine Kompaktheit weiter verbessert werden. Vorzugsweise sind die erste Übertragungsrichtung und die zweite Übertragungsrichtung zueinander antiparallel. Die erste Übertragungsrichtung ist insbesondere eine proximale Richtung. Unter einer "proximalen Richtung" soll insbesondere eine Richtung verstanden werden, welche in Richtung eines Benutzers der Vorrichtung ausgerichtet ist. Die zweite Übertragungsrichtung ist insbesondere eine distale Richtung. Unter einer "distalen Richtung" soll insbesondere eine Richtung verstanden werden, welche in Richtung eines zu behandelnden Patienten ausgerichtet ist.

Es wird zudem vorgeschlagen, dass das Ultraschallhorn sich entlang der ersten Übertragungsrichtung verjüngt. Hierdurch kann vorteilhaft eine Kompaktheit weiter verbessert werden, da derart das Ultraschallhorn weniger Bauraum innerhalb eines Gehäuses benötigt. Auch können hierdurch Ultraschallwellen besser fokussiert werden. Das Ultraschallhorn weist vorzugsweise eine sich insbesondere konkav verjüngende Zylinderform auf.

Um vorteilhaft einen Bauraum weiter zu verringern wird vorgeschlagen, dass das Ultraschallhorn die Sonotrode koaxial umgebend angeordnet ist. Darunter, dass "zwei Bauteile koaxial angeordnet sind", soll insbesondere verstanden werden, dass beide Bauteile eine identische Haupterstreckungsachse aufweisen. Die Sonotrode weist einen Außendurchmesser auf, welcher im Wesentlichen kleiner ist als ein Außendurchmesser des Ultraschallhorns. Unter dem Ausdruck "im Wesentlichen kleiner" soll insbesondere verstanden werden wenigstens um 10% kleiner, vorzugsweise um wenigstens 20% kleiner und besonders bevorzugt um wenigstens 30% kleiner.

In einer Ausgestaltung der Erfindung wird vorgeschlagen, dass die Sonotrode das Ultraschallhorn entlang der zweiten Übertragungsrichtung durchstößt. Hierdurch kann vorteilhaft ein Bauraum weiter reduziert werden. Das Ultraschallhorn weist insbesondere eine zu der Sonotrode korrespondierend ausgebildete Ausnehmung auf, innerhalb welcher die Sonotrode zumindest teilweise angeordnet ist.

Um eine effiziente Energieübertragung zu gewährleisten und trotzdem wenig Bauraum, insbesondere innerhalb eines Gehäuses der Lithotripsievorrichtung, zu beanspruchen, wird des Weiteren vorgeschlagen, dass eine Haupterstreckung der Sonotrode größer ist als eine Haupterstreckung des Ultraschallhorns. Insbesondere ragt ein Teil der Sonotrode distalseitig über das Ultraschallhorn hinaus. Besonders bevorzugt ragt die Ultraschallspitze über das Ultraschallhorn hinaus. Insbesondere liegt der Sonotrodenkopf innerhalb eines Bereichs des Ultraschallhorns. Die Haupterstreckung der Sonotrode ist insbesondere zumindest um 15%, vorzugsweise zumindest um 30% und besonders bevorzugt zumindest um 45% größer als eine Haupterstreckung des Ultraschallhorns.

Des Weiteren wird vorgeschlagen, dass die Lithotripsievorrichtung eine Koppeleinheit umfasst, welche zu einer Beaufschlagung der Sonotrode dazu eingerichtet ist, die Sonotrode mit einer Stoßimpulseinheit zu koppeln. Es kann vorteilhaft ein modularer Aufbau erzielt werden, wobei insbesondere verschiedene Komponenten der Lithotripsievorrichtung abhängig von einer Betriebsart miteinander koppelbar sind. Unter einer "Koppeleinheit" soll insbesondere eine Einheit verstanden werden, welche dazu eingerichtet ist, zumindest zwei weitere Bauteile miteinander wirkverbunden zu koppeln. Dazu kann die Koppeleinheit zu einer form- und/oder kraftschlüssigen Verbindung eingerichtet sein. Insbesondere weist die Lithotripsievorrichtung die Stoßimpulseinheit auf. Unter einer "Stoßimpulseinheit" soll insbesondere eine Einheit verstanden werden, welche zur Bereitstellung von Stoßimpulsen wenigstens ein beweglich gelagertes Projektil umfasst, welches dazu eingerichtet ist, die Sonotrode zumindest mittelbar mit Stoßimpulsen zu beaufschlagen. Die Stoßimpulseinheit ist insbesondere dazu eingerichtet, das Projektil mechanisch, pneumatisch, hydraulisch und/oder elektromagnetisch zu beschleunigen, wobei vorzugsweise das Projektil sein durch die Beschleunigung erfahrenen Impuls zumindest teilweise, vorzugsweise zu einem Großteil und besonders bevorzugt vollständig als Stoßimpuls auf die Sonotrode überträgt.

Um eine bauteileffiziente Kopplung zu erzielen wird insbesondere vorgeschlagen, dass die Koppeleinheit zumindest ein Übertragungselement aufweist, welches zumindest eine erste Wirkfläche aufweist, die dazu eingerichtet ist, einen Impuls aufzunehmen und wenigstens eine zweite Wirkfläche aufweist, welche dazu eingerichtet ist, den aufgenommenen Impuls auf die Sonotrode entlang der zweiten Übertragungsrichtung zumindest mittelbar zu übertragen. Die erste Wirkfläche ist insbesondere eine proximale Wirkfläche. Die erste Wirkfläche zeigt vorzugsweise in Richtung der ersten Übertragungsrichtung. Die erste Wirkfläche ist insbesondere dazu eingerichtet, einen Stoßimpuls zumindest mittelbar von der Stoßimpulseinheit aufzunehmen und zwar insbesondere von dem Projektil der Stoßimpulseinheit. Insbesondere ist die zweite Wirkfläche eine distale Wirkfläche. Die zweite Wirkfläche zeigt vorzugsweise in Richtung der zweiten Übertragungsrichtung. Die zweite Wirkfläche ist insbesondere dazu eingerichtet, einen Stoßimpuls zumindest mittelbar auf die Sonotrode und zwar insbesondere den Sonotrodenkopf zu übertragen. Die erste Wirkfläche und die zweite Wirkfläche sind zueinander, vorzugsweise entgegengesetzt ausgerichtet. Das Übertragungselement ist insbesondere separat von der Sonotrode ausgebildet. Alternativ ist es denkbar, dass das Übertragungselement zumindest teilweise einstückig mit der Sonotrode ausgebildet ist. Beispielsweise könnte das Übertragungselement einstückig mit dem Sonotrodenkopf ausgebildet sein. Das Übertragungselement ist insbesondere zwischen dem Sonotrodenkopf und dem Projektil der Stoßimpulseinheit angeordnet. Besonders bevorzugt ist das Übertragungselement als ein Bolzen ausgebildet.

Weiterhin wird vorgeschlagen, dass die Koppeleinheit einen Schnellverbinder umfasst, welcher zu einer herstell- und/oder lösbaren mechanischen Verbindung der Stoßimpulseinheit mit der Sonotrode eingerichtet ist. Hierdurch kann vorteilhaft eine Kopplung zwischen Stoßimpulseinheit und der Sonotrode vereinfacht werden. Unter einem "Schnellverbinder" soll insbesondere eine Einheit verstanden werden, welche zu einer einfachen und/oder schnellen, vorzugsweise werkzeuglosen und/oder einhändigen Herstellung und/oder Aufhebung einer Verbindung zumindest zwei der Bauteile eingerichtet ist. Der Schnellverbinder kann insbesondere einen Schnellspanner und vorzugsweise einen Bajonettverschluss umfassen. Ferner ist der Schnellverbinder zu einer austauschbaren Verbindung von Bauteilen vorgesehen. Unter einer "austauschbaren Verbindung" soll insbesondere eine Verbindung zwischen einem ersten Bauteil und einem zweiten Bauteil verstanden werden, wobei das erste Bauteil gegen zumindest ein weiteres zusätzliches Bauteil ausgetauscht werden kann, welches insbesondere an Stelle des ersten Bauteils mit dem zweiten Bauteil verbindbar ist. Beispielsweise ist denkbar, dass verschieden ausgebildete Stoßimpulseinheiten mit der Sonotrode koppelbar sind. Beispielsweise könnte man eine pneumatische Stoßimpulseinheit, eine mechanische Stoßimpulseinheit, eine elektromagnetische Stoßimpulseinheit und/oder eine hydraulische Stoßimpulseinheit variabel verbunden werden, um Stoßimpulse auf die Sonotrode zu übertragen.

Ferner wird vorgeschlagen, dass die Lithotripsievorrichtung eine Fluideinheit umfasst, die wenigstens einen Fluidkanal umfasst, welcher wenigstens teilweise von der Sonotrode ausgebildet ist und wenigstens eine Abwinklung aufweist. Unter einem "Fluidkanal" soll insbesondere eine Baueinheit verstanden werden, die zumindest teilweise zu einer Führung eines Fluidstroms vorgesehen ist und die den Fluidstrom, in einer Hauptströmungsrichtung betrachtet, unmittelbar zumindest teilweise, vorzugsweise auf drei Seiten und besonders vorteilhaft vollständig umschließt. Vorzugsweise ist eine Erstreckung des Fluidkanals entlang der Hauptströmungsrichtung des Fluidstroms zumindest 1-mal, insbesondere wenigstens 3-mal und vorteilhaft zumindest 5-mal so lang wie wenigstens eine Querschnittserstreckung an einem Punkt des Fluidkanals senkrecht zu der Hauptströmungsrichtung des Fluidstroms an dem Punkt. Unter einem "Fluid" soll in diesem Zusammenhang insbesondere eine Flüssigkeit, vorzugsweise Wasser, ein Gas und/oder ein Gas-Flüssigkeits-Gemisch verstanden werden. Unter einer "Abwinklung" soll insbesondere ein Zwischenabschnitt des Fluidkanals verstanden werden, entlang dessen eine Hauptströmungsrichtung des Fluidkanals variiert. Vorzugsweise bildet der Zwischenabschnitt zumindest eine Biegung oder einen Knick aus. Insbesondere weist der Fluidkanal zumindest einen ersten Teilabschnitt und zumindest einen zweiten Teilabschnitt auf, welche insbesondere zueinander winklig ausgerichtet sind. Insbesondere ist der Zwischenabschnitt vorzugsweise unmittelbar zwischen dem ersten Teilabschnitt und dem zweiten Teilabschnitt angeordnet. Vorteilhaft weist der erste Teilabschnitt eine erste Haupterstreckungsrichtung und/oder eine erste Hauptströmungsrichtung auf und der zweite Teilabschnitt weist zumindest eine zweite Haupterstreckungsrichtung und/oder zumindest eine zweite Hauptströmungsrichtung auf, wobei die erste Haupterstreckungsrichtung und/oder die erste Hauptströmungsrichtung winklig zur zweiten Haupterstreckungsrichtung und/oder zur zweiten Hauptströmungsrichtung ausgerichtet ist. Unter "winklig ausgerichtet" soll in diesem Zusammenhang insbesondere von parallel und/oder antiparallel verschieden ausgerichtet verstanden werden. Vorzugsweise schließt der erste Teilabschnitt, insbesondere die erste Haupterstreckungsrichtung und/oder die erste Hauptströmungsrichtung, mit dem zweiten Teilabschnitt, insbesondere der zweiten Haupterstreckungsrichtung und/oder der zweiten Hauptströmungsrichtung, des Fluidkanals zumindest einen Winkel von zumindest 45°, insbesondere von zumindest 60°, vorteilhaft von zumindest 75° und besonders bevorzugt von zumindest im Wesentlichen 90° ein. Unter einem Winkel von "zumindest im Wesentlichen 90" soll insbesondere ein Winkel von
90° insbesondere mit einer Abweichung von höchstens 8°, vorzugsweise von höchstens 5° und besonders bevorzugt von höchstens 2° verstanden werden. Insbesondere sind die erste Haupterstreckungsrichtung und/oder die erste Hauptströmungsrichtung zumindest im Wesentlichen parallel zu der Haupterstreckungsrichtung der Sonotrode. Insbesondere sind die zweite Haupterstreckungsrichtung und/oder die zweite Hauptströmungsrichtung zumindest im Wesentlichen senkrecht zu der Haupterstreckungsrichtung der Sonotrode.

Des Weiteren wird vorgeschlagen, dass der Fluidkanal zumindest teilweise von der Koppeleinheit ausgebildet ist. Es kann vorteilhaft ein Bauraum eingespart werden. Insbesondere ist die Abwinklung des Fluidkanals im Bereich der Koppeleinheit angeordnet ist. Vorzugsweise ist die Abwinklung in das Übertragungselement der Koppeleinheit integriert. Das Übertragungselement weist insbesondere eine Ausnehmung auf, welche die Abwinklung ausbildet.

Zudem vorgeschlagen, dass die Lithotripsievorrichtung wenigstens eine Lagereinheit umfasst, welche dazu eingerichtet ist, die Sonotrode und/oder das Übertragungselement beweglich entlang der ersten und/oder zweiten Übertragungsrichtung schwimmend zu lagern. Es kann vorteilhaft ein Eintrag von Stoßimpulsen in die Sonotrode verbessert werden. Die Lagereinheit ist insbesondere als ein Gleitlager ausgebildet. Die Lagereinheit ist insbesondere von aneinander liegenden Oberflächen der Sonotrode und/oder das Übertragungselement sowie eines in das Gehäuse eingelassenen Führungskanal gebildet.

Es wird zudem vorgeschlagen, dass die Lagereinheit zumindest ein Dichtelement umfasst, welches wenigstens teilweise den Fluidkanal begrenzt. Es kann vorteilhaft weitere Bauteile eingespart werden.

Ferner wird vorgeschlagen, dass das Dichtelement mit der Sonotrode und dem Übertragungselement verbunden ist. Es kann vorteilhaft weitere Bauteile welche zu einer Abdichtung sowohl der Sonotrode als auch des Übertragungselement notwendig wären eingespart werden. Vorzugsweise ist das Dichtelement kraft- und/oder formschlüssig mit der Sonotrode und dem Übertragungselement verbunden.

Das Dichtelement könnte beispielsweise als ein O-Ring ausgebildet sein. In einer offenbarungsgemäßen Ausgestaltung wird vorgeschlagen, dass das Dichtelement als ein Faltenbalg ausgebildet ist. Es kann vorteilhaft weitere Bauteile eingespart werden

Ein weiterer Aspekt der Erfindung geht aus von einem Verfahren zum Betrieb einer Lithotripsievorrichtung, wie beispielsweise zu einem vorzugsweise extrakorporalen Testbetrieb einer Lithotripsievorrichtung, insbesondere einer solchen Lithotripsievorrichtung, bei welchem Ultraschallwellen von einem Sonotrodenkopf einer Sonotrode aufgenommen wird und Konkrementen von wenigstens einer Sonotrodenspitze der Sonotrode, welche mit dem Sonotrodenkopf verbunden ist, beaufschlagt werden und die Ultraschallwellen von einem Ultraschallhorn weitergeleitet werden.

Es wird vorgeschlagen, dass das Ultraschallhorn die Ultraschallwellen entlang einer ersten Übertragungsrichtung zumindest mittelbar auf den Sonotrodenkopf überträgt und fokussiert und die Sonotrode entlang wenigstens einer zweiten Übertragungsrichtung, die von der ersten Übertragungsrichtung verschieden ist, die Ultraschallwellen vom Sonotrodenkopf zur Sonotrodenspitze überträgt.

Hierdurch kann vorteilhaft eine Effizienz verbessert werden. Insbesondere kann eine kompakte Anordnung von Bauteilen realisiert werden, wodurch sich weitere Baugruppen und Komponenten eines Lithotripters, wie insbesondere eine Stoßimpulseinheit, zur kombinierten Lithotripsie einbinden lassen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung eines Lithotripsiesystems mit einer Lithotripsievorrichtung in einer perspektivischen Ansicht,
- Fig. 2: eine schematische Darstellung eines Teils der Lithotripsievorrichtung in einer perspektivischen Ansicht,
- Fig. 3: eine schematische Darstellung eines Teils der Lithotripsievorrichtung in einer Schnittansicht,
- Fig. 4: eine schematische Darstellung eines Teils der Lithotripsievorrichtung mit einer Koppeleinheit in einer Schnittansicht,
- Fig. 5: einen schematischen Ablaufplan eines beispielhaften Verfahrens zum Betrieb der Lithotripsievorrichtung und
- Fig. 6: eine schematische Darstellung einer alternativen Ausgestaltung einer Lithotripsievorrichtung mit einer alternativen Lithotripsievorrichtung.

### Beschreibung der Ausführungsbeispiele

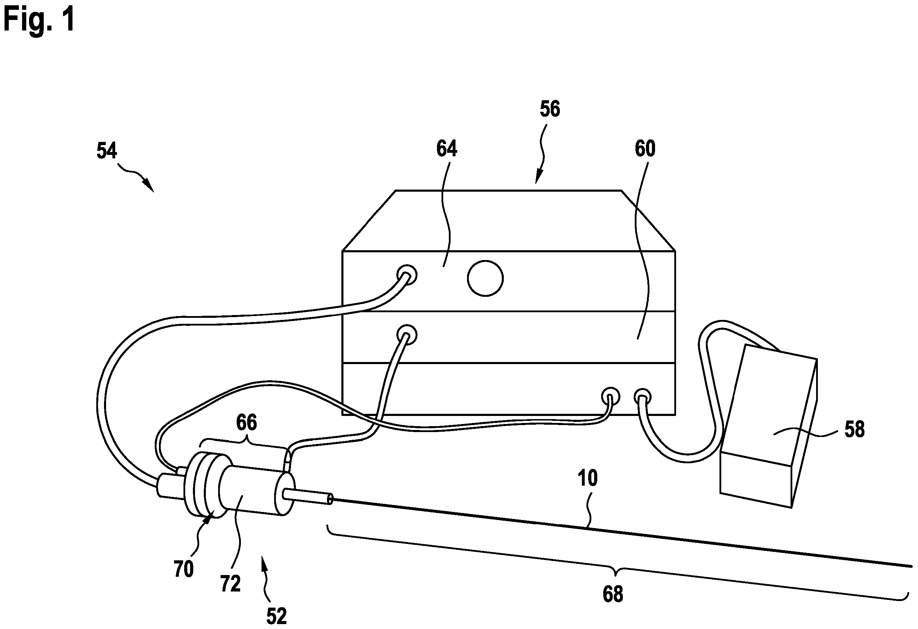

Fig. 1 zeigt eine schematische Darstellung eines Lithotripsiesystems 54 in einer perspektivischen Ansicht. Das Lithotripsiesystem 54 weist wenigstens einen Lithotripter 52 auf. Der Lithotripter 52 ist im vorliegenden Fall als ein intrakorporaler Lithotripter 52 ausgebildet. Alternativ könnte ein Lithotripter auch als ein extrakorporaler Lithotripter ausgebildet sein.

Der Lithotripter 52 weist wenigstens eine Lithotripsievorrichtung auf. Im vorliegenden Fall bildet die Lithotripsievorrichtung den Lithotripter 52 vollständig aus. Alternativ könnte die Lithotripsievorrichtung nur einen Teil eines Lithotripters ausbilden.

Das Lithotripsiesystem 54 weist wenigstens ein Steuergerät 56 auf. Das Steuergerät 56 ist zumindest zu einer Steuerung der Lithotripsievorrichtung eingerichtet. Die Lithotripsievorrichtung ist mit dem Steuergerät 56 verbunden. Das Steuergerät 56 besitzt zumindest eine Steuerelektronik (nicht dargestellt). Zu einer Ansteuerung der Lithotripsievorrichtung ist die Steuerelektronik elektrisch und/oder elektronisch mit dieser verbunden. Die Steuerelektronik enthält zumindest einen Prozessor. Ferner enthält die Steuerelektronik wenigstens einen Speicher. Auf dem Speicher ist ein Betriebsprogramm hinterlegt. Das Betriebsprogramm ist von dem Prozessor ausführbar.

Das Lithotripsiesystem 54 weist wenigstens ein Betätigungsmittel 58 auf. Das Betätigungsmittel 58 ist zu einer Aktivierung und/oder Deaktivierung der Lithotripsievorrichtung ausgebildet. Das Betätigungsmittel 58 ist mit dem Steuergerät 56 und zwar insbesondere mit der Steuerelektronik des Steuergeräts 56 verbunden. Im vorliegenden Fall ist das Betätigungsmittel 58 als ein Fußschalter ausgebildet. Alternativ könnte ein Betätigungsmittel auch als ein anderer elektrischer und/oder elektronischer Schalter ausgebildet sein, wie beispielsweise als ein Druckknopf, einen Kippschalter oder dergleichen, welcher insbesondere Teil einer Lithotripsievorrichtung sein könnte. Auch könnte eine Bedienung der Lithotripsievorrichtung über ein externes Gerät, insbesondere ein Handgerät, wie beispielsweise ein Tablet, ein Smartphone, eine Smartwatch oder dergleichen erfolgen.

Das Lithotripsiesystem 54 weist ferner eine Fördereinrichtung 60 auf. Die Fördereinrichtung 60 ist im vorliegenden Fall als eine Pumpe ausgebildet. Die Fördereinrichtung 60 ist zur Förderung eines Irrigats eingerichtet. Dazu ist die Fördereinrichtung 60 fluidtechnisch mit der Lithotripsievorrichtung verbunden. Die Fördereinrichtung 60 dient zum Absaugen eines Irrigats und/oder zu einem Freispulen mittels eines Irrigats bei einem Lithotripsievorgang. Bei dem Irrigat kann es sich um eine Flüssigkeit handeln, wie in etwa eine Kochsalzlösung. Beispielsweise können mit dem Irrigat Bruchteile eines bei einem Lithotripsievorgang zerstörten Konkrements abtransportiert werden.

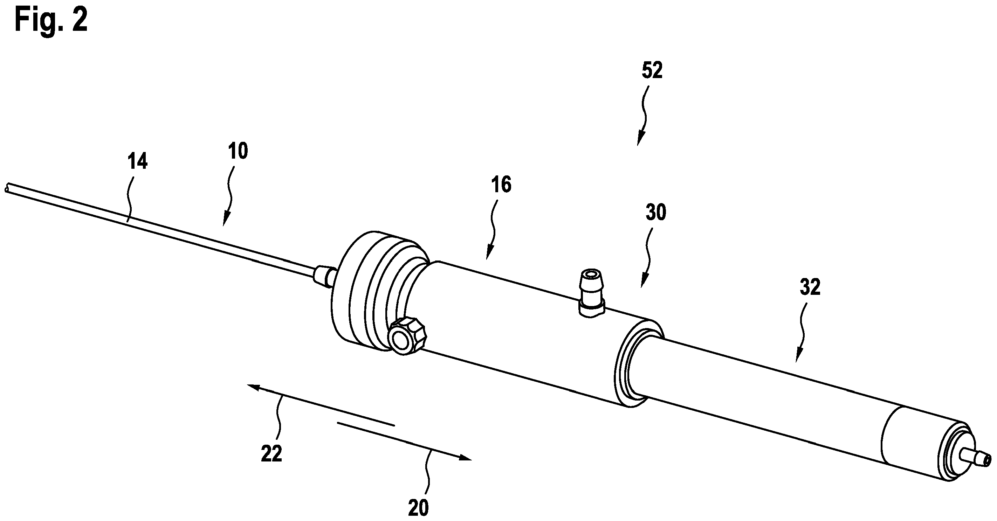

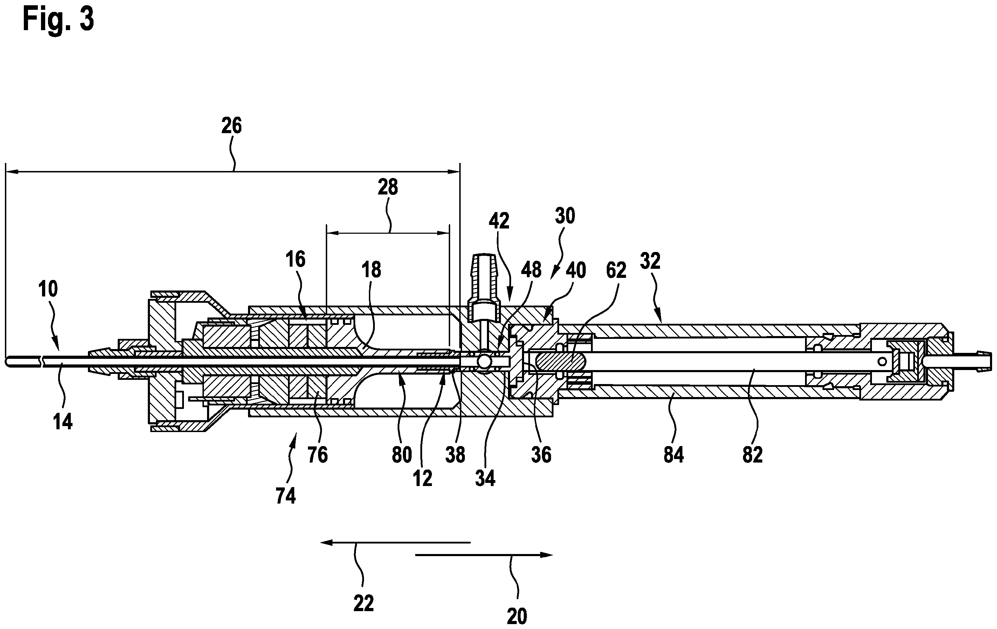

Zur Erzeugung von Stoßimpulsen, wie beispielsweise mittels eines Projektils 62, der Lithotripsievorrichtung ist wenigstens eine Antriebseinrichtung 64 vorgesehen (vgl. Fig. 2 und 3). Die Antriebseinrichtung 64 ist im vorliegenden Fall separat von der Lithotripsievorrichtung ausgebildet. Die Antriebseinrichtung 64 ist Teil des Lithotripsiesystems 54. Die Antriebseinrichtung 64 ist Teil des Steuergeräts 56. Die Antriebseinrichtung 64 ist im vorliegenden Fall als eine pneumatische Antriebseinrichtung 64 ausgebildet. Die Antriebseinrichtung 64 ist als ein Druckluftgenerator ausgebildet. Alternativ könnte es sich bei der Antriebseinrichtung um eine mechanische, elektromagnetische oder eine hydraulische Antriebseinrichtung handeln. Alternativ könnte eine solche Antriebseinrichtung auch Teil einer solchen Lithotripsievorrichtung sein, beispielsweise, wenn diese Antriebseinrichtung als eine elektromagnetische Antriebseinheit, wie in etwa ein linearer Aktor. Auch könnte eine Antriebeinrichtung ein von einem Steuergerät separates Antriebsgerät ausbilden.

Die Lithotripsievorrichtung besitzt einen proximalen Abschnitt 66. Der proximale Abschnitt 66 ist bei einer Bedienung einem Bediener der Lithotripsievorrichtung zugewandt. Der proximale Abschnitt 66 ist bei einer Bedienung einem Patienten abgewandt. Der proximale Abschnitt 66 liegt bei einer Behandlung eines Patienten außerhalb des Patienten. Ferner besitzt die Lithotripsievorrichtung einen distalen Abschnitt 68. Der distale Abschnitt 68 ist bei einer Bedienung einem Bediener der Lithotripsievorrichtung abgewandt. Der distale Abschnitt 68 ist bei einer Bedienung einem Patienten zugewandt. Der distale Abschnitt 68 liegt bei einer Behandlung eines Patienten zumindest teilweise innerhalb des Patienten.

Die Lithotripsievorrichtung weist ein Gehäuse 70 auf. Das Gehäuse 70 ist hohlzylinderförmig. Das Gehäuse 70 bildet zumindest teilweise den proximalen Abschnitt 66 der Lithotripsievorrichtung aus. Das Gehäuse 70 dient zur Anordnung weiterer Komponenten der Lithotripsievorrichtung.

Ferner weist die Lithotripsievorrichtung eine Handhabe 72 auf. Die Handhabe 72 ist zu einer manuellen Bedienung der Lithotripsievorrichtung ausgebildet. Die Handhabe 72 ist von dem Gehäuse 70 ausgebildet. Die Handhabe 72 bildet zumindest teilweise den proximalen Abschnitt 66a aus. Alternativ könnte insbesondere bei einer robotischen Ansteuerung einer solchen Lithotripsievorrichtung auch auf eine Handhabe verzichtet werden oder zu einer Anbindung an einen Roboterarm genutzt werden.

Fig. 2 zeigt eine schematische Darstellung eines Teils der Lithotripsievorrichtung in einer perspektivischen Ansicht und Fig. 3 in einer Schnittansicht.

Die Lithotripsievorrichtung weist wenigstens einen Ultraschallgenerator 74 auf. Der Ultraschallgenerator 74 ist dazu ausgebildet, Ultraschallwellen bereitzustellen. Zu einer Ansteuerung ist der Ultraschallgenerator 74 mit dem Steuergerät 56 und zwar insbesondere der Steuerelektronik verbunden. Der Ultraschallgenerator 74 ist zumindest teilweise in dem Gehäuse 70 angeordnet. Im vorliegenden Fall ist der Ultraschallgenerator 74 vollständig in dem Gehäuse 70 angeordnet. Der Ultraschallgenerator 74 bildet zumindest teilweise den proximalen Abschnitt 66 der Lithotripsievorrichtung aus.

Der Ultraschallgenerator 74 enthält wenigstens einen Ultraschallaktor 76. Im vorliegenden Fall weist der Ultraschallgenerator 74 mehrere Ultraschallaktoren 76 auf. Der Ultraschallgenerator 74 weist zwei Ultraschallaktoren 76 auf. Die Ultraschallaktoren 76 sind aneinander anliegend gestapelt angeordnet. Der Übersichtlichkeit halber ist in den Zeichnungen nur ein Ultraschallaktor 76 mit einem Bezugszeichen versehen. Die Ultraschallaktoren 76 sind zumindest im Wesentlichen identisch zueinander ausgebildet. Im Folgenden ist nur ein Ultraschallaktor 76 näher beschrieben. Diese Beschreibung ist auch auf die weiteren Ultraschallaktoren 76 übertragbar. Im vorliegenden Fall ist der Ultraschallaktor 76 als ein Piezoaktor ausgebildet. Der Ultraschallaktor 76 ist zylinderplattenförmig ausgebildet. Der Übersichtlichkeit halber sind etwaige Anschluss- und/oder Kontaktierungsmittel und/oder Isolationen der Ultraschallaktoren 76 nicht in der Fig. 2 bzw. 3 dargestellt.

Ferner weist die Lithotripsievorrichtung wenigstens ein Ultraschallhorn 18 auf. Das Ultraschallhorn 18 nimmt die von dem Ultraschallgenerator 74 bereitgestellten Ultraschallwellen auf. Das Ultraschallhorn 18 ist zur Übertragung von Ultraschallwellen ausgebildet. Das Ultraschallhorn 18 liegt an dem Ultraschallaktor 76 an.

Das Ultraschallhorn 18 ist zumindest teilweise in dem Gehäuse 70 angeordnet. Das Ultraschallhorn 18 bildet zumindest teilweise den proximalen Abschnitt 66 der Lithotripsievorrichtung aus. Das Ultraschallhorn 18 ist zumindest teilweise außerhalb des Gehäuse 70 angeordnet. Das Ultraschallhorn 18 weist die Form eines Rotationskörpers auf. Im vorliegenden Fall weist das Ultraschallhorn 18 eine hohlzylinderartige Form auf. Das Ultraschallhorn 18 ist zumindest teilweise aus Metall, insbesondere Stahl oder Titan ausgebildet. Der Ultraschallgenerator 74 liegt an dem Ultraschallhorn 18 an.

Das Ultraschallhorn 18 verjüngt sich entlang der ersten Übertragungsrichtung 20. Das Ultraschallhorn 18 weist eine Verjüngung auf. Die Verjüngung liegt innerhalb des Gehäuses 70. Die Verjüngung ist frei von Stufen. Die Verjüngung weist einen stetigen Verlauf auf. Der Verlauf der Verjüngung ist konkav. Der Verlauf der Verjüngung ist exponentiell. Alternativ oder zusätzlich könnte die Verjüngung auch stufenförmig oder katenoidalförmig sein. Das Ultraschallhorn 18 weist eine Ausnehmung 80 auf. Die Ausnehmung 80 verläuft koaxial entlang einer Rotationsachse des Ultraschallhorns 18.

Die Lithotripsievorrichtung weist wenigstens eine Sonotrode 10 auf. Die Sonotrode 10 weist eine Haupterstreckung 26 auf. Die Sonotrode 10 ist zumindest teilweise außerhalb des Gehäuse 70 angeordnet. Die Sonotrode 10 bildet zumindest zu einem Großteil den distalen Abschnitt 68 der Lithotripsievorrichtung aus. Die Sonotrode 10 ist rohrförmig ausgebildet. Die Sonotrode 10 ist starr ausgebildet. Die Sonotrode 10 besteht zumindest teilweise aus Metall, insbesondere Stahl. Zur Aufnahme von Ultraschallwellen weist die Sonotrode 10 wenigstens einen Sonotrodenkopf 12 auf. Der Sonotrodenkopf 12 bildet zumindest teilweise ein proximales End der Sonotrode 10 aus. Zur Beaufschlagung von Konkrementen weist die Sonotrode 10 wenigstens eine Sonotrodenspitze 14 auf. Die Sonotrodenspitze 14 bildet zumindest teilweise ein distales Ende der Sonotrode 10 aus. Die Sonotrodenspitze 14 ist mit dem Sonotrodenkopf 12 verbunden.

Die Sonotrode 10 ist zumindest teilweise in/an dem Ultraschallhorn 18 angeordnet. Die Sonotrode 10 ist von dem Ultraschallhorn 18 umgeben. Das Ultraschallhorn 18 ist die Sonotrode 10 koaxial umgebend angeordnet. Die Sonotrode 10 ist in der Ausnehmung 80 des Ultraschallhorns 18 angeordnet. Die Sonotrode 10 weist eine Haupterstreckung 26 auf. Das Ultraschallhorn 18 weist eine Haupterstreckung 28 auf. Die Haupterstreckung 26 der Sonotrode 10 ist größer als die Haupterstreckung 28 des Ultraschallhorns 18. Die Sonotrode 10 und die Ultraschallaktoren 76 sind so zueinander angeordnet, dass die Haupterstreckung 28 des Ultraschallhorn 18 vollständig innerhalb der Haupterstreckung 26 der Sonotrode 10 liegt.

Das Ultraschallhorn 18 ist dazu eingerichtet, entlang einer ersten Übertragungsrichtung 20 Ultraschallwellen zumindest mittelbar auf einen Sonotrodenkopf 12 der Sonotrode 10 zu übertragen und zu fokussieren. Die erste Übertragungsrichtung 20 entspricht einer proximalen Richtung. Die Sonotrode 10a ist mit dem Ultraschallhorn 18 verbunden. Der Sonotrodenkopf 12 ist mit dem Ultraschallhorn 18 verbunden. Die Sonotrode 10 und das Ultraschallhorn 18 sind im vorliegenden Fall miteinander verschraubt. Alternativ oder zusätzlich kann die Sonotrode mit dem Ultraschallhorn verklebt oder verlötet sein. Ferner ist denkbar, dass eine solche Sonotrode mit solch einem Ultraschallhorn einstückig ausgebildet sein könnte.

Die Sonotrode 10 ist dazu eingerichtet, entlang wenigstens einer zweiten Übertragungsrichtung 22, die von der ersten Übertragungsrichtung 20 verschieden ist, die Ultraschallwellen vom Sonotrodenkopf 12 zur Sonotrodenspitze 14 zu übertragen. Die zweite Übertragungsrichtung 22 entspricht einer distalen Richtung. Die erste Übertragungsrichtung 20 und die zweite Übertragungsrichtung 22 sind einander entgegengerichtet. Die Sonotrode 10 durchstößt das Ultraschallhorn 18 entlang der zweiten Übertragungsrichtung 22. Die Sonotrode 10 ragt proximalseitig aus dem Ultraschallhorn 18 heraus. Ferner ragt sie Sonotrode 10 distalseitig aus den Ultraschallwellen heraus.

Zusätzlich zu einer Behandlung mit Ultraschallwellen ist die Lithotripsievorrichtung dazu eingerichtet, Stoßimpulse auf Konkremente zu übertragen. Dazu weist die Lithotripsievorrichtung eine Stoßimpulseinheit 32 auf. Die Stoßimpulseinheit 32 wenigstens ein Projektil 62 auf. Das Projektil 62 ist linear beweglich gelagert. In wenigstens einem Betriebszustand ist das Projektil 62 dazu ausgebildet, die Sonotrode 10 und/oder das Ultraschallhorn 18 zumindest mittelbar zu beaufschlagen. Im vorliegenden Fall beaufschlagt das Projektil 62 die Sonotrode 10 mittelbar. Zur Lagerung des Projektils 62 weist die Stoßimpulseinheit 32 wenigstens einen Führungskanal 82 auf. Die Lithotripsievorrichtung weist ein Führungsrohr 84 auf, welches den Führungskanal 82 begrenzt. Alternativ ist es denkbar, dass das Projektil innerhalb der Sonotrode oder des Ultraschallhorns beweglich gelagert sein könnte.

Die Antriebseinrichtung 64 ist mit der Stoßimpulseinheit 32 gekoppelt. Die die Stoßimpulseinheit 32 ist mit dem Führungskanal 82 verbunden. An dem Führungsrohr 84 ist ein Verbindungsstutzen angeordnet. Die Antriebseinrichtung 64a ist über einen Schlauch mit dem Verbindungsstutzen verbunden. Die Antriebseinrichtung 64a überträgt Druckluftstöße auf den Führungskanal 82, wodurch das in dem Führungskanal 82 angeordnete Projektil 62a beschleunigt wird. Die Antriebseinrichtung 64a beschleunigt das Projektil 62a innerhalb des Führungskanals 82.

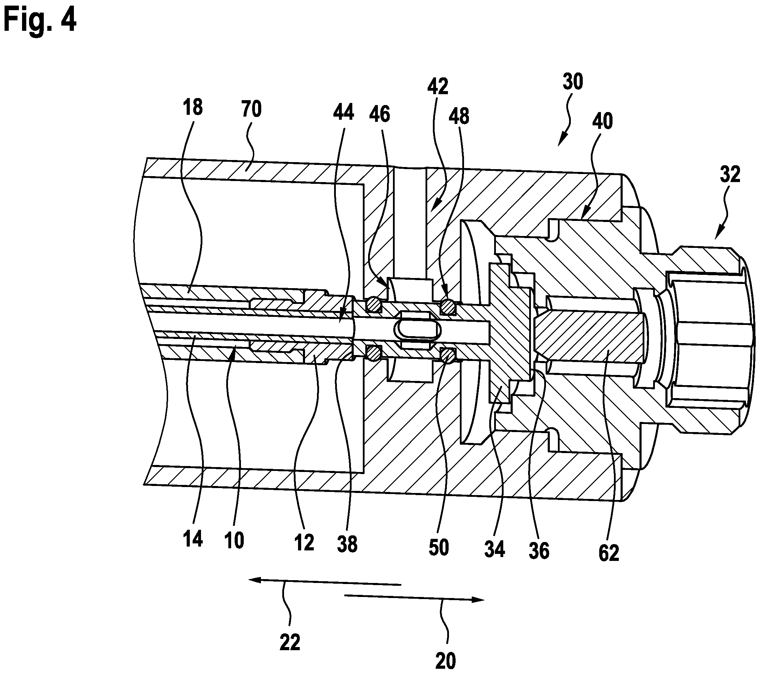

Die Lithotripsievorrichtung weist eine Koppeleinheit 30 auf (siehe Fig. 4). Die Koppeleinheit 30 ist dazu eingerichtet, die Sonotrode 10 mit der Stoßimpulseinheit 32 zu koppeln. Die Koppeleinheit 30 weist zumindest ein Übertragungselement 34 auf. Das Übertragungselement 34 weist zumindest eine erste Wirkfläche 36 auf. Die erste Wirkfläche 36 ist dazu eingerichtet, einen Impuls aufzunehmen. In einem Betriebszustand schlägt das Projektil 62 auf die Wirkfläche 36. Ferner weist das Übertragungselement 34 zumindest eine zweite Wirkfläche 38 auf. Die zweite Wirkfläche 38 liegt der ersten Wirkfläche 36 gegenüber. Die zweite Wirkfläche 38 ist dazu eingerichtet, den aufgenommenen Impuls auf die Sonotrode 10 entlang der zweiten Übertragungsrichtung 22 zumindest mittelbar zu übertragen. Das Übertragungselement 34 ist schwimmend gelagert. Dazu ist das Übertragungselement 34 unter Spiel zwischen der Sonotrode zur 14 und dem Projektil 62 angeordnet. Es ist denkbar, dass das Übertragungselement auch einstückig mit der Sonotrode 10 ausgebildet sein könnte.

Die Koppeleinheit 30 weist einen Schnellverbinder 40 auf. Der Schnellverbinder 40 ist zu einer herstell- und lösbaren mechanischen Verbindung der Stoßimpulseinheit 32 mit der Sonotrode 10 eingerichtet. Der Schnellverbinder 40 ist dazu eingerichtet, eine werkzeuglos und zerstörungsfrei lösbare Verbindung herzustellen. Im vorliegenden Fall ist die Verbindung eine kraft- und/oder formschlüssige Verbindung. Der Schnellverbinder 40 bildet im vorliegenden Fall den Schraubverschluss aus. Alternativ sind noch andere Schnellverbinderarten denkbar, wie beispielsweise einen Bajonettverschluss oder dergleichen.

Die Lithotripsievorrichtung weist eine Fluideinheit 42 auf. Die Fluideinheit 42 ist dazu eingerichtet, ein Fluid zum Spülen bereitzustellen und/oder ein Fluid abzuführen. Die Fluideinheit 42 weist wenigstens einen Fluidkanal 44 auf. Der Fluidkanal 44 ist zumindest teilweise von der Sonotrode 10 ausgebildet. Die Sonotrode 10 als eine axiale Ausnehmung auf, welche den Fluidkanal 44 teilweise ausbildet. Der Fluidkanal 44 ist zumindest teilweise von der Koppeleinheit 30 ausgebildet. Das Übertragungselement 34 weist eine axiale Ausnehmung auf, welche den Fluidkanal 44 teilweise ausbildet.

Der Fluidkanal 44 weist wenigstens eine Abwinklung 46 auf. Die Abwinklung 46 des Fluidkanals 44 ist im Bereich der Koppeleinheit 30 angeordnet. Die Abwinklung 46 ist in das Übertragungselement 34 integriert. Das Übertragungselement 34 weist wenigstens eine radiale Öffnung auf. Die radiale Öffnung stellt eine Verbindung zu der axialen Ausnehmung des Übertragungselements 34 her.

Die Lithotripsievorrichtung weist wenigstens eine Lagereinheit 48 auf. Die Lagereinheit 48 ist dazu eingerichtet, die Sonotrode 10 beweglich entlang der ersten und/oder zweiten Übertragungsrichtung 20, 22 schwimmend zu lagern. Die Lagereinheit 48 ist als ein Gleitlager ausgebildet. Die Lagereinheit 48 weist zumindest ein Dichtelement 50 auf. Die Lagereinheit 48 ist von aneinander liegenden Oberflächen der Sonotrode 10 und/oder das Übertragungselement 34 sowie eines in das Gehäuse 70 eingelassenen Führungskanal 82 gebildet.

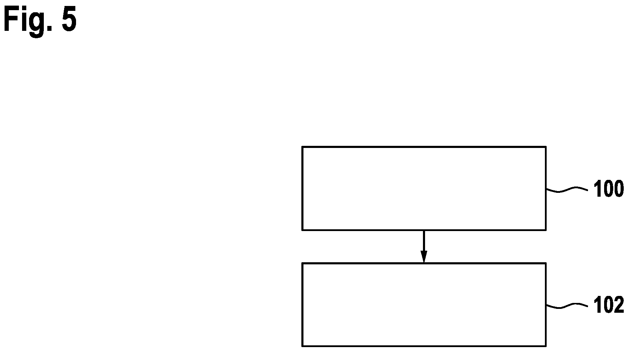

Figur 5 zeigt einen schematischen Ablaufplan eines beispielhaften Verfahrens zum Betrieb der Lithotripsievorrichtung. Bei dem Betrieb handelt es sich im vorliegenden Fall um einen extrakorporalen Testbetrieb.

Das Verfahren umfasst einen Verfahrensschritt 100. In dem Verfahrensschritt 100 wird der Ultraschallgenerator 74 aktiviert. Der Ultraschallgenerator 74 erzeugt Ultraschallwellen. Die Ultraschallwellen werden von dem Ultraschallhorn 18 entlang der ersten Übertragungsrichtung 20 übertragen und fokussiert. Die Ultraschallwellen werden von dem Sonotrodenkopf 12 aufgenommen. Von dem Sonotrodenkopf 12 aus werden die Ultraschallwellen entlang einer zweiten Übertragungsrichtung 22 auf den Sonotrodenspitze 14 übertragen. Die Sonotrodenspitze 14 überträgt die Ultraschallwellen wiederum auf etwaige Konkremente, welche hierdurch zerstört werden können.

Das Verfahren umfasst ein Verfahrensschritt 102. In dem Verfahrensschritt 102 wird die Stoßimpulseinheit 32 aktiviert. Die Antriebseinrichtung 64 beschleunigt das Projektil 62 pneumatisch. Das Projektil 62 schlägt am Ende einer Beschleunigungsstrecke auf das Übertragungselement 34. Dadurch wird eine Bewegung des Projektils 62 größtenteils gestoppt und ein Stoßimpuls des Projektils 62 an das Übertragungselement 34 abgegeben. Da das Übertragungselement 34 in Kontakt mit der Sonotrode 10 steht, überträgt diese wiederum den Stoßimpuls an den Sonotrodenkopf 12. Der Stoßimpuls wird entlang der zweiten Übertragungsrichtung 22 von dem Sonotrodenkopf 12 auf die Sonotrodenspitze 14 übertragen. Die Sonotrodenspitze 14 überträgt den Stoßimpuls wiederum auf etwaige Konkremente, welche hierdurch zerstört werden können.

Die Verfahrensschritte 100, 102 können dabei in beliebiger Reihenfolge alternierend oder aber auch gleichzeitig durchgeführt werden. Gerade bei der gleichzeitigen Ausführung der Verfahrensschritte 100 und 102 kann ein Kombinationsbetrieb erzielt werden, bei welchen etwaige Konkrementen gleichzeitig mit Ultraschallwellen und Stoßimpulsen beaufschlagt werden können.

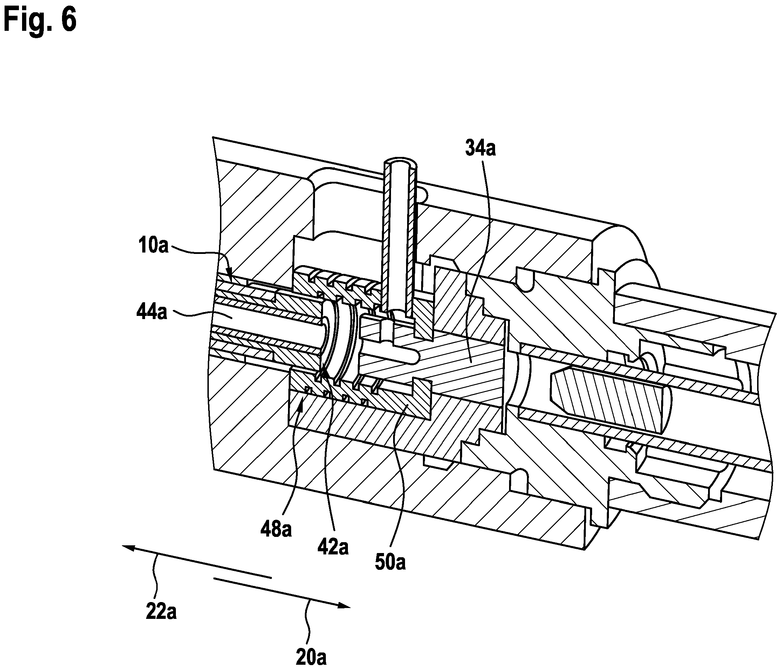

In der Figur 6 ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 5 verwiesen wird. Zur Unterscheidung der Ausführungsbeispiele ist der Buchstabe a den Bezugszeichen dem Ausführungsbeispiel der Figur 6 nachgestellt.

Die Lithotripsievorrichtung weist wenigstens eine Lagereinheit 48a auf. Die Lagereinheit 48a ist dazu eingerichtet, eine Sonotrode 10a der Lithotripsievorrichtung beweglich entlang einer ersten und/oder einer zweiten Übertragungsrichtung 20a, 22a schwimmend zu lagern. Die Lagereinheit 48a weist zumindest ein Dichtelement 50a auf, welches wenigstens teilweise einen Fluidkanal 44a einer Fluideinheit 42a der Lithotripsievorrichtung begrenzt. Das Dichtelement 50a ist mit der Sonotrode 10a und einem Übertragungselement 34a einer Koppeleinheit 30a der Lithotripsievorrichtung verbunden. Das Dichtelement 50a ist als ein Faltenbalg ausgebildet ist. Dichtelement 50a ist kraft- und/oder formschlüssig mit einer Sonotrode 10a und dem Übertragungselement 34a verbunden.

| | | | |
|---|---|---|---|
| 10 | Sonotrode | 60 | Fördereinrichtung |
| 12 | Sonotrodenkopf | 62 | Projektil |
| 14 | Sonotrodenspitze | 64 | Antriebseinrichtung |
| 16 | Ultraschalleinheit | 66 | Proximaler Abschnitt |
| 18 | Ultraschallhorn | 68 | Distaler Abschnitt |
| 20 | erste Übertragungsrichtung | 70 | Gehäuse |
| 22 | zweite Übertragungsrichtung | 72 | Handhabe |
| 26 | Haupterstreckung der Sonotrode | 74 | Ultraschallgenerator |
| 28 | Haupterstreckung des Ultraschallhorns | 76 | Ultraschallaktor |
| | | 80 | Ausnehmung des Ultraschallhorns |
| 30 | Koppeleinheit | | |
| 32 | Stoßimpulseinheit | 82 | Führungskanal |
| 34 | Übertragungselement | 84 | Führungsrohr |
| 36 | Erste Wirkfläche | 100 | Verfahrensschritt |
| 38 | Zweite Wirkfläche | 102 | Verfahrensschritt |
| 40 | Schnellverbinder | | |
| 42 | Fluideinheit | | |
| 44 | Fluidkanal | | |
| 46 | Abwinklung | | |
| 48 | Lagereinheit | | |
| 50 | Dichtelement | | |
| 52 | Lithotripter | | |
| 54 | Lithotripsiesystem | | |
| 56 | Steuergerät | | |
| 58 | Betätigungsmittel | | |

## Patentansprüche

1. Lithotripsievorrichtung, insbesondere intrakorporale Lithotripsievorrichtung, mit wenigstens einer Sonotrode (10a), welche zur Aufnahme von Ultraschallwellen wenigstens einen Sonotrodenkopf (12) und zur Beaufschlagung von Konkrementen wenigstens eine Sonotrodenspitze (14), welche mit dem Sonotrodenkopf (12) verbunden ist, umfasst und mit einer Ultraschalleinheit (16), welche wenigstens ein Ultraschallhorn (18) aufweist, wobei die Sonotrode (10a) dazu eingerichtet ist, entlang wenigstens einer zweiten Übertragungsrichtung (22, 22a) die Ultraschallwellen vom Sonotrodenkopf (12) zur Sonotrodenspitze (14) zu übertragen, **dadurch gekennzeichnet, dass** das Ultraschallhorn (18) sich entlang einer ersten Übertragungsrichtung (20, 20a) verjüngt und dazu eingerichtet ist, entlang der ersten Übertragungsrichtung (20, 20a) Ultraschallwellen zumindest mittelbar auf den Sonotrodenkopf (12) zu übertragen und zu fokussieren, wobei die zweite Übertragungsrichtung (22, 22a) von der ersten Übertragungsrichtung (20, 20a) verschieden ist.

2. Lithotripsievorrichtung nach Anspruch 1, **dadurch gekennzeichnet, dass** die erste Übertragungsrichtung (20, 20a) und die zweite Übertragungsrichtung (22, 22a) einander entgegengerichtet sind.

3. Lithotripsievorrichtung nach Anspruch 1 oder 2, **dadurch gekennzeichnet, dass** das Ultraschallhorn (18) die Sonotrode (10a) koaxial umgebend angeordnet ist.

4. Lithotripsievorrichtung nach einem der vorhergehenden Ansprüche, **dadurch gekennzeichnet, dass** die Sonotrode (10a) das Ultraschallhorn (18) entlang der zweiten Übertragungsrichtung (22, 22a) durchstößt.

5. Lithotripsievorrichtung nach einem der vorhergehenden Ansprüche, **dadurch gekennzeichnet, dass** eine Haupterstreckung (26) der Sonotrode (10a) größer ist als eine Haupterstreckung (28) des Ultraschallhorns (18).

6. Lithotripsievorrichtung nach einem der vorhergehenden Ansprüche, **gekennzeichnet durch** eine Koppeleinheit (30a), welche zu einer Beaufschlagung der Sonotrode (10a) dazu eingerichtet ist, die Sonotrode (10a) mit einer Stoßimpulseinheit (32) zu koppeln.

7. Lithotripsievorrichtung nach Anspruch 6, **dadurch gekennzeichnet, dass** die Koppeleinheit (30a) zumindest ein Übertragungselement (34a) aufweist, welches zumindest eine erste Wirkfläche (36) aufweist, die dazu eingerichtet ist, einen Impuls aufzunehmen, und wenigstens eine zweite Wirkfläche (38), welche der ersten Wirkfläche (36) gegenüber liegt, aufweist, welche dazu eingerichtet ist, den aufgenommenen Impuls auf die Sonotrode (10a) entlang der zweiten Übertragungsrichtung (22, 22a) zumindest mittelbar zu übertragen.

8. Lithotripsievorrichtung nach Anspruch 6 oder 7, **dadurch gekennzeichnet, dass** die Koppeleinheit (30a) einen Schnellverbinder (40) umfasst, welcher zu einer herstell- und lösbaren mechanischen Verbindung der Stoßimpulseinheit (32) mit der Sonotrode (10a) eingerichtet ist.

9. Lithotripsievorrichtung nach einem der vorhergehenden Ansprüche, **gekennzeichnet durch** eine Fluideinheit (42a), die wenigstens einen Fluidkanal (44a) umfasst, welcher wenigstens teilweise von der Sonotrode (10a) ausgebildet ist und wenigstens eine Abwinklung (46) aufweist.

10. Lithotripsievorrichtung nach einem der Ansprüche 6 bis 8 und nach Anspruch 9, **dadurch gekennzeichnet, dass** der Fluidkanal (44a) zumindest teilweise von der Koppeleinheit (30a) ausgebildet ist.

11. Lithotripsievorrichtung nach Anspruch 9 oder 10, **dadurch gekennzeichnet, dass** die Abwinklung (46) des Fluidkanals (44a) im Bereich der Koppeleinheit (30a) angeordnet ist.

12. Lithotripsievorrichtung nach einem der Ansprüche 7 oder 8 und nach einem der Ansprüche 9 bis 11, **dadurch gekennzeichnet, dass** die Abwinklung (46) in das Übertragungselement (34a) integriert ist.

13. Lithotripsievorrichtung nach einem der vorhergehenden Ansprüche, **gekennzeichnet durch** wenigstens eine Lagereinheit (48a), welche dazu eingerichtet ist, die Sonotrode (10a) beweglich entlang der ersten und/oder zweiten Übertragungsrichtung (20, 20a, 22, 22a) schwimmend zu lagern.

14. Lithotripsievorrichtung nach einem der Ansprüche 9 bis 12 und nach Anspruch 13, **dadurch gekennzeichnet, dass** die Lagereinheit (48a) zumindest ein Dichtelement (50a) umfasst, welches wenigstens teilweise den Fluidkanal (44a) begrenzt.

15. Lithotripsievorrichtung nach einem der Ansprüche 7 oder 8 und nach Anspruch 14, **dadurch gekennzeichnet, dass** das Dichtelement (50a) mit der Sonotrode (10a) und dem Übertragungselement (34a) verbunden ist.

16. Lithotripsievorrichtung zumindest nach Anspruch 14 oder 15, **dadurch gekennzeichnet, dass** das Dichtelement (50a) als ein Faltenbalg ausgebildet ist.

17. Lithotripter (52), insbesondere intrakorporaler Lithotripter, mit wenigstens einer Lithotripsievorrichtung nach einem der vorhergehenden Ansprüche.

18. Lithotripsiesystem (54) mit wenigstens einem Lithotripter (52) nach Anspruch 17 und mit wenigstens einem Steuergerät (56), welches zur Steuerung des Lithotripters (52) gerichtet ist.

19. Verfahren zum extrakorporalen Testbetrieb einer Lithotripsievorrichtung, insbesondere einer Lithotripsievorrichtung nach einem der Ansprüche 1 bis 16, bei welchem Ultraschallwellen von einem Sonotrodenkopf (12) einer Sonotrode (10a) aufgenommen wird und Konkrementen von wenigstens einer Sonotrodenspitze (14) der Sonotrode (10a), welche mit dem Sonotrodenkopf (12) verbunden ist, beaufschlagt werden und die Ultraschallwellen von einer Ultraschalleinheit (16), welche wenigstens ein Ultraschallhorn (18) aufweist, bereitgestellt werden, wobei das Ultraschallhorn (18) die Ultraschallwellen entlang einer ersten Übertragungsrichtung (20, 20a) zumindest mittelbar auf den Sonotrodenkopf (12) überträgt und fokussiert und die Sonotrode (10a) entlang wenigstens einer zweiten Übertragungsrichtung (22, 22a), die von der ersten Übertragungsrichtung (20, 20a) verschieden ist, die Ultraschallwellen vom Sonotrodenkopf (12) zur Sonotrodenspitze (14) überträgt, wobei das Ultraschallhorn (18) sich entlang der ersten Übertragungsrichtung (20, 20a) verjüngt.

## Claims

1. Lithotripsy device, in particular an intracorporeal lithotripsy device, comprising at least one sonotrode (10a) which comprises at least one sonotrode head (12) for receiving ultrasonic waves and at least one sonotrode tip (14) for acting upon concretions, which sonotrode tip is connected to the sonotrode head (12), and comprising an ultrasound unit (16) which has at least one ultrasonic horn (18), the sonotrode (10a) being designed to transmit the ultrasonic waves from the sonotrode head (12) to the sonotrode tip (14) along at least one second transmission direction (22, 22a),**characterized in that** the ultrasonic horn (18) tapers along a first transmission direction (20, 20a) and is designed to transmit and focus ultrasonic waves at least indirectly onto the sonotrode head (12) along the first transmission direction (20, 20a), the second transmission direction (22, 22a) differing from the first transmission direction (20, 20a).

2. Lithotripsy device according to claim 1, **characterized in that** the first transmission direction (20, 20a) and the second transmission direction (22, 22a) are opposite to one another.

3. Lithotripsy device according to claim 1 or claim 2, **characterized in that** the ultrasonic horn (18) is arranged so as to coaxially surround the sonotrode (10a).

4. Lithotripsy device according to any of the preceding claims, **characterized in that** the sonotrode (10a) penetrates the ultrasonic horn (18) along the second transmission direction (22, 22a).

5. Lithotripsy device according to any of the preceding claims, **characterized in that** a main extension (26) of the sonotrode (10a) is greater than a main extension (28) of the ultrasonic horn (18).

6. Lithotripsy device according to any of the preceding claims, **characterized by** a coupling unit (30a) which, in order to act upon the sonotrode (10a), is designed to couple the sonotrode (10a) to a shock pulse unit (32).

7. Lithotripsy device according to claim 6, **characterized in that** the coupling unit (30a) has at least one transmission element (34a) which has at least one first active surface (36) which is designed to receive a pulse, and at least one second active surface (38) which is opposite the first active surface (36) and which is designed to transmit the received pulse at least indirectly to the sonotrode (10a) along the second transmission direction (22, 22a).

8. Lithotripsy device according to claim 6 or claim 7, **characterized in that** the coupling unit (30a) comprises a quick connector (40) which is designed to provide a mechanical connection, that can be established and released, between the shock pulse unit (32) and the sonotrode (10a).

9. Lithotripsy device according to any of the preceding claims, **characterized by** a fluid unit (42a) comprising at least one fluid channel (44a) which is formed, at least in part, by the sonotrode (10a) and which has at least one bend (46).

10. Lithotripsy device according to any of claims 6 to 8 and according to claim 9, **characterized in that** the fluid channel (44a) is formed, at least in part, by the coupling unit (30a).

11. Lithotripsy device according to claim 9 or claim 10, **characterized in that** the bend (46) of the fluid channel (44a) is arranged in the region of the coupling unit (30a).

12. Lithotripsy device according to either claim 7 or claim 8 and according to any of claims 9 to 11, **characterized in that** the bend (46) is integrated into the transmission element (34a).

13. Lithotripsy device according to any of the preceding claims, **characterized by** at least one support structure (48a) which is designed to support the sonotrode (10a) in a floating manner such that the sonotrode is movable along the first and/or the second transmission direction (20, 20a, 22, 22a).

14. Lithotripsy device according to any of claims 9 to 12 and according to claim 13, **characterized in that** the support structure (48a) comprises at least one sealing element (50a) which at least partially delimits the fluid channel (44a).

15. Lithotripsy device according to either claim 7 or claim 8 and according to claim 14, **characterized in that** the sealing element (50a) is connected to the sonotrode (10a) and the transmission element (34a).

16. Lithotripsy device at least according to claim 14 or claim 15, **characterized in that** the sealing element (50a) is formed as a bellows.

17. Lithotripter (52), in particular an intracorporeal lithotripter, comprising at least one lithotripsy device according to any of the preceding claims.

18. Lithotripsy system (54) comprising at least one lithotripter (52) according to claim 17 and comprising at least one control unit (56) for controlling the lithotripter (52).

19. Method for extracorporeal test operation of a lithotripsy device, in particular of a lithotripsy device according to any of claims 1 to 16, in which ultrasonic waves are received by a sonotrode head (12) of a sonotrode (10a) and concretions are acted upon by at least one sonotrode tip (14) of the sonotrode (10a), which sonotrode tip is connected to the sonotrode head (12), and the ultrasonic waves are provided by an ultrasound unit (16) which has at least one ultrasonic horn (18), wherein the ultrasonic horn (18) transmits and focuses the ultrasonic waves at least indirectly onto the sonotrode head (12) along a first transmission direction (20, 20a) and the sonotrode (10a) transmits the ultrasonic waves from the sonotrode head (12) to the sonotrode tip (14) along at least one second transmission direction (22, 22a) which is different from the first transmission direction (20, 20a), wherein the ultrasonic horn (18) tapers along the first transmission direction (20, 20a).

## Revendications

1. Dispositif de lithotripsie, en particulier dispositif de lithotripsie intracorporelle, comportant au moins une sonotrode (10a), laquelle comprend au moins une tête de sonotrode (12) pour la réception d'ondes ultrasonores, et au moins une pointe de sonotrode (14), laquelle est reliée à la tête de sonotrode (12), pour la sollicitation de concrétions, et comportant une unité à ultrasons (16) qui présente au moins un cornet à ultrasons (18), dans lequel la sonotrode (10a) est configurée pour transmettre les ondes ultrasonores de la tête de sonotrode (12) à la pointe de sonotrode (14), le long d'au moins une seconde direction de transmission (22, 22a), **caractérisé en ce que** le cornet à ultrasons (18) se rétrécit le long d'une première direction de transmission (20, 20a) et est configuré pour transmettre et focaliser des ondes ultrasonores au moins indirectement sur la tête de sonotrode (12) le long de la première direction de transmission (20, 20a), dans lequel la seconde direction de transmission (22, 22a) est différente de la première direction de transmission (20, 20a).

2. Dispositif de lithotripsie selon la revendication 1, **caractérisé en ce que** la première direction de transmission (20, 20a) et la seconde direction de transmission (22, 22a) sont opposées l'une à l'autre.

3. Dispositif de lithotripsie selon la revendication 1 ou 2, **caractérisé en ce que** le cornet à ultrasons (18) est disposé de manière à entourer de manière coaxiale la sonotrode (10a).

4. Dispositif de lithotripsie selon l'une des revendications précédentes, **caractérisé en ce que** la sonotrode (10a) perce le cornet à ultrasons (18) le long de la seconde direction de transmission (22, 22a).

5. Dispositif de lithotripsie selon l'une des revendications précédentes, **caractérisé en ce qu'une** extension principale (26) de la sonotrode (10a) est supérieure à une extension principale (28) du cornet à ultrasons (18).

6. Dispositif de lithotripsie selon l'une des revendications précédentes, **caractérisé par** une unité de couplage (30a) qui, afin de solliciter la sonotrode (10a), est configurée pour coupler la sonotrode (10a) à une unité d'impulsion d'impact (32).

7. Dispositif de lithotripsie selon la revendication 6, **caractérisé en ce que** l'unité de couplage (30a) présente au moins un élément de
transmission (34a) qui présente au moins une première surface active (36) qui est configurée pour recevoir une impulsion, et au moins une seconde surface active (38) qui est opposée à la première surface active (36) et qui est configurée pour transmettre au moins indirectement l'impulsion reçue à la sonotrode (10a) le long de la seconde direction de transmission (22, 22a).

8. Dispositif de lithotripsie selon la revendication 6 ou 7, **caractérisé en ce que** l'unité de couplage (30a) comprend une liaison rapide (40) qui est conçue pour une liaison mécanique réalisable et amovible de l'unité d'impulsion d'impact (32) avec la sonotrode (10a).

9. Dispositif de lithotripsie selon l'une des revendications précédentes, **caractérisé par** une unité fluidique (42a) qui comprend au moins un canal fluidique (44a) qui est réalisé au moins partiellement par la sonotrode (10a) et présente au moins un coude (46).

10. Dispositif de lithotripsie selon l'une des revendications 6 à 8 et selon la revendication 9, **caractérisé en ce que** le canal fluidique (44a) est réalisé au moins partiellement par l'unité de couplage (30a).

11. Dispositif de lithotripsie selon la revendication 9 ou 10,
**caractérisé en ce que** le coude (46) du canal fluidique (44a) est disposé dans la zone de l'unité de couplage (30a).

12. Dispositif de lithotripsie selon l'une des revendications 7 ou 8 et selon l'une des revendications 9 à 11, **caractérisé en ce que** le coude (46) est intégré à l'élément de transmission (34a).

13. Dispositif de lithotripsie selon l'une des revendications précédentes, **caractérisé par** au moins une unité de soutien (48a) qui est conçue pour soutenir de manière flottante la sonotrode (10a) de manière à permettre son déplacement le long de la première et/ou de la seconde direction de transmission (20, 20a, 22, 22a).

14. Dispositif de lithotripsie selon l'une des revendications 9 à 12 et selon la revendication 13, **caractérisé en ce que** l'unité de soutien (48a) comprend au moins un élément d'étanchéité (50a) qui délimite au moins partiellement le canal fluidique (44a).

15. Dispositif de lithotripsie selon l'une des revendications 7 ou 8 et selon la revendication 14, **caractérisé en ce que** l'élément d'étanchéité (50a) est relié à la sonotrode (10a) et à l'élément de transmission (34a).

16. Dispositif de lithotripsie au moins selon la revendication 14 ou 15, **caractérisé en ce que** l'élément d'étanchéité (50a) est réalisé sous forme de soufflet.

17. Lithotripteur (52), en particulier lithotripteur intracorporel, comportant au moins un dispositif de lithotripsie selon l'une des revendications précédentes.

18. Système de lithotripsie (54) comportant au moins un lithotripteur (52) selon la revendication 17 et comportant au moins un appareil de commande (56) qui est configuré pour commander le lithotripteur (52).

19. Procédé permettant le fonctionnement d'essai extracorporel d'un dispositif de lithotripsie, en particulier d'un dispositif de lithotripsie selon l'une des revendications 1 à 16, dans lequel des ondes ultrasonores sont reçues par une tête de sonotrode (12) d'une sonotrode (10a) et des concrétions sont soumises à au moins une pointe de sonotrode (14) de la sonotrode (10a), laquelle est reliée à la tête de sonotrode (12), et les ondes ultrasonores sont fournies par une unité à ultrasons (16) qui présente au moins un cornet à ultrasons (18), dans lequel le cornet à ultrasons (18) transmet et focalise les ondes ultrasonores au moins indirectement sur la tête de sonotrode (12) le long d'une première direction de transmission (20, 20a) et la sonotrode (10a) transmet les ondes ultrasonores de la tête de sonotrode (12) à la pointe de sonotrode (14) le long d'au moins une seconde direction de transmission (22, 22a), laquelle est différente de la première direction de transmission (20, 20a), dans lequel le cornet à ultrasons (18) se rétrécit le long de la première direction de transmission (20, 20a).
